# EUROPEAN PATENT SPECIFICATION

(11) **EP 3 393 550 B1**
(45) Date of publication and mention of the grant of the patent: **11.12.2019**
(21) Application number: 16834288.9
(22) Date of filing: 22.12.2016
(51) Int. Cl.: A61M 5/158, A61M 5/32, A61M 25/06

(54) **DISPOSABLE SAFETY BUTTERFLY NEEDLE**
WEGWERFBARE SICHERHEITSBUTTERFLYNADEL
AIGUILLE JETABLE À AILETTES DE SÉCURITÉ

(30) Priority: 23.12.2015 IT UB20159438
(43) Date of publication of application: 31.10.2018
(73) Proprietor: BNP S.r.L., 55032 Castelnovo di Garfagnana (IT)
(72) Inventor: NARDINI, Pasquale, 50125 Firenze (IT)
(74) Representative: Valenza, Silvia
(86) International application number: PCT/IB2016/057934
(87) International publication number: WO 2017/109747

(56) References cited:
- EP-A1- 1 323 449
- WO-A1-2004/000396
- US-A1- 2004 167 477

## Description

### FIELD OF THE INVENTION

The present invention relates to the field of medical devices for venipuncture, and in particular to a disposable safety butterfly needle.

### STATE OF THE ART

As is known, butterfly needles for venipuncture are common on the market. Such butterfly needles generally are used for intravenous injections, drips, blood samples, transfusions and the like. Even if specific reference is made later to injections, it is to be understood that the butterfly needle according to the invention also may be used for blood samples and the like.

A butterfly needle generally comprises an actual needle for penetrating the vein. Such a needle is supported integrally by a needle-holder on which two flexible butterfly-shaped wings are mounted.

The rear end of the needle is connected to a small plastic tube. The tube ends with a larger opening, which is closed by a cap. The opening of the tube is intended to be coupled with the head of a syringe filled with solution to be injected for intravenous injections, or with the head of a drip container or bottle.

Such flexible wings perform a dual function: i.e. they serve as a grasp for the insertion into the vein and the extraction from the vein by the operator who should carry out the injection, and as securing means to the patient's skin to block the needle in position during the injection.

Since the butterfly needle - tube - syringe - bottle, etc. assembly may not be reused, it is disposed of. Such an operation is highly dangerous because the butterfly needle remains uncovered and this results in the risk of accidental pricks, both by the user carrying out the injection and the personnel charged with disposal. WO2011/055287 describes a safety butterfly needle comprising a retraction mechanism of the needle in which the needle is protected inside the cylindrical body supporting the flexible wings; said mechanism operating by means of a compressed spring; said mechanism actuated manually by means of a lever actuated by the wings. However, the butterfly needle described in WO2011/055287 had the problem of accidental closing caused by the practice by the medical personnel of using the wings for the grasp between their thumb and index finger, therefore preventing the operator from having maximum freedom of manual movement during the delicate operation of venipuncture. Moreover, this needle has a closing mechanism whereby the needle rotates in the vein before translating. This butterfly needle is therefore not practical. WO2004/000396, US2004/0167477 and EP1323449 also describe prior art safety butterfly needles comprising a mechanism for retracting the needle after use.

It is the object of the present invention to eliminate the drawbacks of the prior art by providing a safety butterfly needle which is capable of avoiding accidental pricks and of making the successive reuse of the apparatus impossible.

It is another object of the present invention to provide a safety butterfly needle which is practical, simple to use for the user and is reliable during use and is safe from accidental closing.

Again another object of the present invention is to provide such a safety butterfly needle which is affordable and simple to make.

### SUMMARY OF THE INVENTION

The present invention achieves the aforesaid objects by means of a safety butterfly needle as defined in independent claim 1.

Advantageous embodiments of the invention are apparent from the dependent claims. In one aspect, the present invention also relates to a method for preparing a butterfly needle as defined in claim 1.

The advantages of the butterfly needle according to the invention are apparent. Indeed, once the injection is carried out, the needle is protected from the body in a safety position, thus avoiding accidental injuries.

Moreover, the functioning of such a butterfly needle appears to be highly safe, precisely because the needle-holder is guided during the axial movement thereof inside the body.

Further features of the invention will be more apparent from the following detailed description, which purely refers to a non-limiting example thereof, which is illustrated in the accompanying drawings.

### BRIEF DESCRIPTION OF THE DRAWINGS

Fig. 1A shows a perspective view (100) and Fig. 1B shows an exploded view of the butterfly needle.
Figures 2A-D show the views (A: front; B: from the top; C: from the back; D from the front) of the half-body (1) and details of certain parts, the body (2) is exactly specular.
Figures 3A-E show the tongue (3) (A: bottom view; B: side view; C: front view) and the rear cap (4) (D: front view; E: view from the bottom) and details of certain parts.
Figures 4A-D show the needle-holder (5) with the needle (6) (A: front view; B: top view) and the safety cap (9) (C: side view; D: front view) and details thereof.
Figures 5A-E show the 5 significant steps of the retraction mechanism of the needle and of the securing:

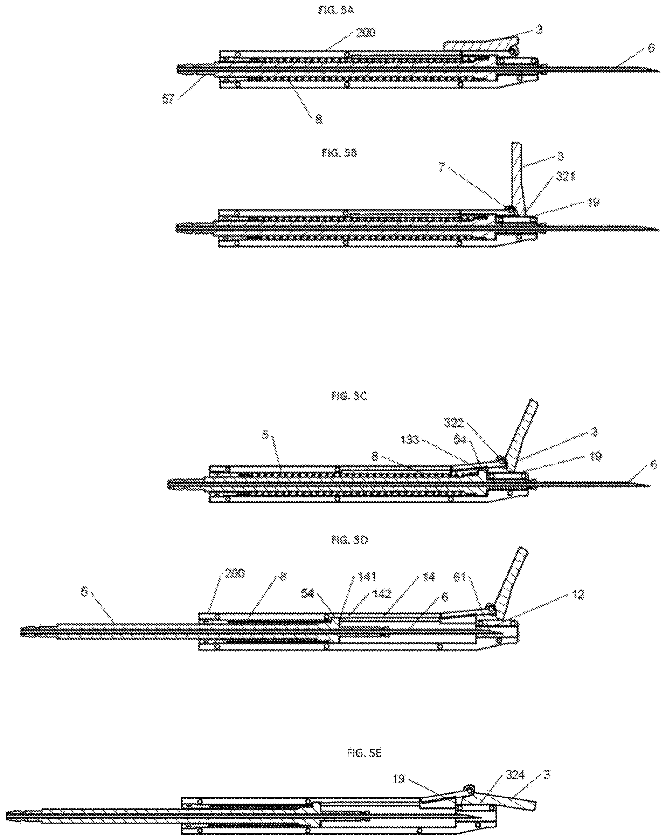

   FIG. 5A device resting or in function, any case operative;
   FIG. 5B preparation for the retraction of the needle;
   FIG. 5C needle retraction trigger;
   FIG. 5D needle retraction;
   FIG. 5E securing.

### DETAILED DESCRIPTION OF THE INVENTION

With the aid of the drawings, the butterfly needle according to the invention indicated as a whole with numeral (100), is now described.

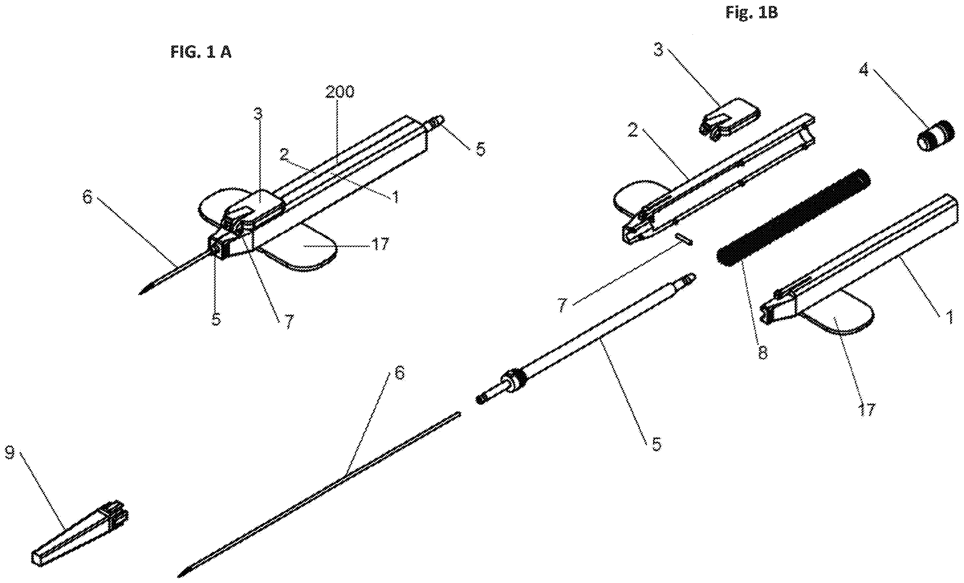

With reference to Fig. 1, the butterfly needle (100) comprises a hollow main body divided into two half-bodies (1 and 2) referring to the whole body (200), having a square section with beveled corners and an axial hole (11), a tongue (3), a rear cap (4), a needle-holder (5), a needle (6), a pivot spine (7), a traction spring (8) and a safety cap (9).

The safety butterfly needle (100) for venipuncture according to the invention comprises a hollow body (200) from which two flexible wings (17) protrude outward, which are foldable by means of a manual grip of the user from a diverging position to a converging position, and a mechanism blocking tongue (3).

A needle (6) is mounted axially in the whole needle-holder (5) coupled to the body (200) so that the needle (6) protrudes axially and frontally from the body (200). The needle-holder (5) has a tail (57) protruding at the rear from the body to be coupled with the coupling of a flexible tube intended to be connected to a syringe or a bottle for injecting a solution or for gathering the blood collected.

The main feature of the invention lies in that the needle-holder (5) is mounted slidingly axially inside the body to pass from an advanced position of use, in which the needle protrudes frontally from the body to carry out the injection, to a retracted safety position in which the needle is protected inside the body (200). The butterfly needle according to the invention further comprises guide means adapted to guide the axial movement of the needle-holder (5) inside the body (200), and stroke-end and blocking means adapted to block the needle-holder in said advanced position of use and in said retracted safety position.

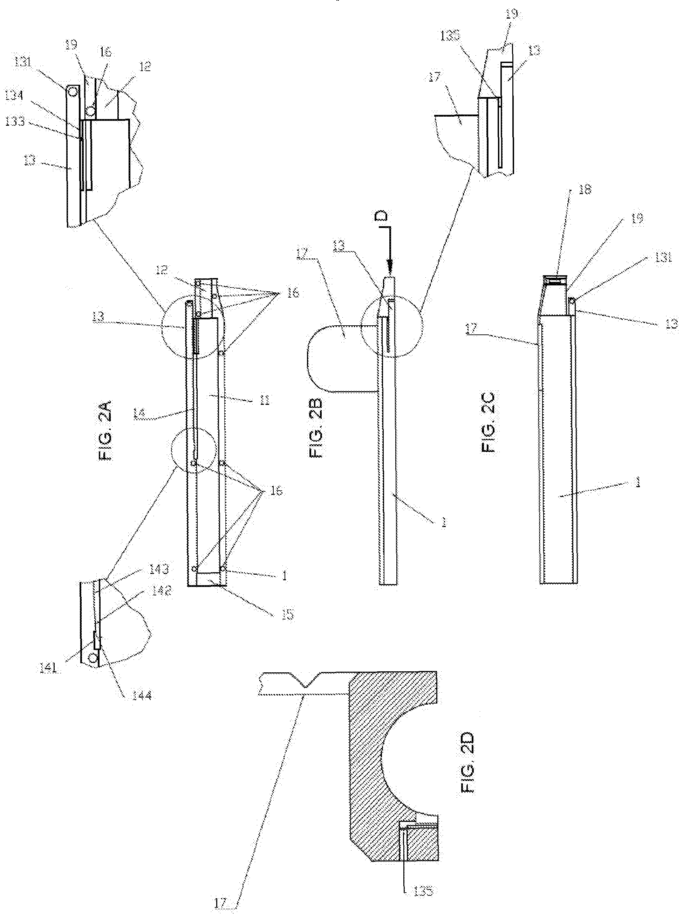

By analyzing in particular the half-body (1) (Fig. 2), and the half-body (2) symmetrically, we can identify a flexible wing (17) and the tongue of the mechanism (13) on the outer part.

Wing (17) is mounted in the front part of the half-body (1); close to the joining between the half-body (1) and wing (17) is a longitudinal groove (171) which acts as fold lines to allow folding. Thereby, by folding along the fold line (171), wing (17) may pass from a flat open position (shown in the figures) to a closed position, in which the wing adheres to the half-body (1) thus achieving an angle of about 90° to provide a suitable grasp to the user.

Looking at body (1), there is the middle hole (11), which may be divided into 4 zones; a middle part (111), the tapered front part (12) which serves to provide stability to the needle-holder (5) until it is extracted from the vein, the guide (14) of nosepiece (54) of the needle-holder and seat (15) for the rear cap (4). Analyzing the guide (14) of nosepiece (54) of the needle-holder (5) (in detail 1.C), it is noted that after the parallel part (143) are a slight ramp (142) and a tooth (144) that results in nosepiece (54) of the needle-holder (5) not coming out of seat (141).

Analyzing the tongue of mechanism (13) (in detail 1.A and 1.B), the pivot hole (131) is found where the pivot spine (7) is inserted with interference which will serve as a pin to tongue (3), the transverse groove (132) allows the release mechanism of the needle-holder (5) to be raised, block (133) serves to block nosepiece (54) of the needle-holder (5) in the housing (134) thereof. When spring (8) is placed in tension, tongue (13) is integral with body (1) beyond to groove (132) by a lamina (135) which will break during the actuation of the mechanism. The edge (19) which will serve to actuate the mechanism is in the front part of body (1), outward above the tapered part (12); on this surface (19) the front part of tongue (13) rests, the one comprising hole (131). Body (1) has, at the interface with the specular half-body (2), the connection holes (16) which couple with the respective pins (26) in the half-body (2) to form body (200). The half-body (1) has, in the front part at the outlet hole of needle (6), groove (18) which serves to block the safety cap (9) by means of tooth (931). The half-body (1) comprises seat (15) for the rear cap (4) at the rear end; said seat (15) has a greater diameter than hole (111).

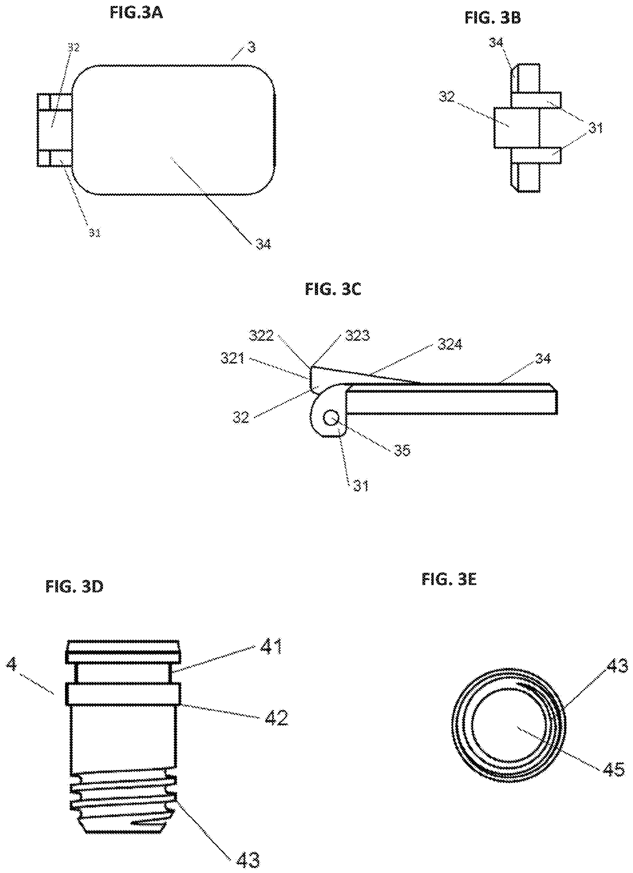

With reference to Fig. 3A, the surface (34) of tongue (3) is noted with the pivot wings (31) with the respective holes (35), where spine (7) enters with some clearance. The essential part of tongue (3) is cam (32) which exerts on edge (19) by actuating the mechanism. Cam (32) is formed by surface (321), the eccentric ramp (322), vertex (323) and surface (324).

With reference to Fig. 3B, noted in the rear cap (4) is groove (41) which is useful for placing spring (8) in tension, abutment (42) which is blocked in seat (15) of the half-body (1), the spiral seat (43) where spring (8) is accommodated and hole (45) which is a guide to the body of the needle-holder (5).

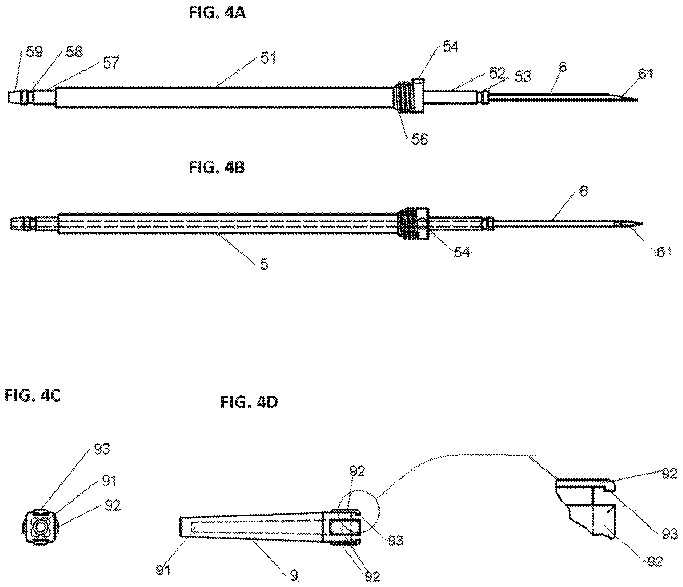

With reference to Fig. 4A, the needle-holder (5) has a body (51) on which spring (8) slides, a tapered front part (52) which is accommodated in the tapered part (12) of the half-body (1); said tapered front part (52) has a groove (53) close to the front end which is useful for placing spring (8) in tension, like groove (41) in the rear cap (4); the nosepiece of the needle-holder (54), which is a cylinder orthogonal to the axis of body (51) of the needle-holder (5), is closed to the front part (52); the spiral seat (56) where spring (8) is accommodated is adjacent to nosepiece (54), in the rear direction; body (51) then has the tapered rear part (57) which protrudes at the rear from the rear cap (4) and serves to attach a flexible tube (not shown in the drawings); the rear part (57) is provided with a groove (58) and a bevel (59) which serve to retain the flexible tube and facilitate the insertion thereof, respectively. The flexible tube is required to connect the butterfly needle (100) to a syringe or a bottle containing the solution to be injected or intended to receive the blood collected. Needle (6) is accommodated in the needle-holder (5) for the entire length, the tip (61) protruding from the hole on the side of the tapered front part (52), the tip (61) is to be placed in step of the nosepiece of the needle-holder (54).

With reference to Fig. 4B, in addition to the capped hole (91) which accommodates the tip of needle (61) and the outer part of the needle-holder (5), the safety cap (9), specifically the tapered front part (52), has 4 wings (92-93). As shown in the detail (9.1), the inward arched wings (93) opposing one another have a tooth (931) which blocks the safety cap (9) to groove (18) on the half-body (1).

Fig. 5 depicts the sectional views of the butterfly needle (100) in the 5 steps of extraction from the vein.

The butterfly needle (100) is in the operating position in Fig. 5A, with needle (6) in position outside body (200) without safety cap (9), therefore hypothetically inside the vein. Spring (8) is tensioned. Tongue (3) adheres to body (200). The tapered rear part (57) alone, the housing of the flexible tube, comes out of the rear part of body (200).

The butterfly needle is in position with the mechanism "armed" in Fig. 5B because tongue (3) was placed at 90° on edge (19) by resting on the surface of cam (321). The tongue pivots on spine (7).

The butterfly needle (100) is in the position in which the mechanism was triggered in Fig. 5C and Fig. 5D. By levering on edge (19) by means of eccentric (322), tongue (3) has broken the 2 laminas (135) and has freed the nosepiece of the needle-holder (54) from block (133), thus allowing spring (8) to return to the resting position by bringing back the entire needle-holder (5) together with needle (6). During the return to the resting position of spring (8), the nosepiece of the needle-holder (54) travels guide (14) while passing ramp (142) and accommodating itself permanently in seat (141) so that the tip of the needle (61) is completely in body (200), precisely in the front tapered part (12).

Tongue (3) in Fig. 5E has completed its travel, thus causing the mechanism not to be rearmed by moving the surface of cam (324) close to edge (19).

The release mechanism of the spring (tongue of mechanism (13) with holes (131) in which plug (7) is accommodated about which tongue (3) rotates) not only may be positioned at the head of the needle-cannula, but also in retracted position along the main body of the apparatus, obviously again in a compatible manner with the compressibility of spring (8).

## Claims

1. A disposable safety butterfly needle comprising a mechanism for retracting the needle (6), wherein the needle is completely retracted inside a hollow body (200) supporting the flexible wings (17); said retraction carried out by a tensioned elastic means (8); said mechanism actuated manually by means of movement of a tongue (3) on a pivot pin (7) which anchors the tongue (3) to the body (200); a movement which generates the break of at least one easy-to-break means (135) of the body (200) whereby the mechanism can no longer be rearmed after having been actuated; wherein said needle (6) is assembled on a needle-holder (5) which slides axially inside the body (200), the needle-holder associated integrally with the front end of the elastic means (8); **characterised in that** said body (200) has, in the forward middle upper part, a mechanism tongue (13) resting on a front outer upper edge (19) of the body (200), said mechanism tongue (13) externally and frontally supporting two holes (131) for accommodating the pivot pin (7) and internally supporting a housing (134) with a blocking guard (133) and a transverse groove at the rear of the housing (134) which allows raising the mechanism tongue (13); said mechanism tongue being integral with the body (200) by means of the groove and by means of at least one lamina (135), which breaks as the mechanism tongue (13) is raised;
wherein said housing (134) holds a nosepiece (54) of the needle-holder (5) by means of the blocking guard (133), said nosepiece (54) being a cylinder orthogonal to the axis of the needle-holder (5), said nosepiece (54) and blocking guard (133) operating as blocking means of the needle-holder in advanced position.

2. A butterfly needle according to claim 1, wherein said hollow main body (200) has a square outer surface with beveled corners and an inner axial hole (11); said body supporting two wings (17) protruding outward and foldable by means of manual grip; wherein said needle-holder (5) is accommodated in the hole (11); said body (200) having, in the rear part, fixing means for the rear end of the elastic means (8); said body (200) having guide means adapted to guide the axial movement of said needle-holder (5) inside said hollow body (200), and blocking and stroke-end means adapted to block said needle-holder in an advanced position of use before the actuation of the mechanism, and in a retracted safety position after the actuation of the mechanism.

3. A butterfly needle according to any one of claims 1-2, wherein the tongue (3) comprises a surface (34) having, at the front end thereof and protruding below the surface (34), the pivot wings (31) with the respective holes (35) where the pivot pin (7) is accommodated; said tongue (3) having, between the wings (31) and protruding above the surface (34), the cam (32) which will operate on the edge (19) of the body (200) by actuating the needle retraction mechanism; the cam (32) comprising a surface (321) orthogonal to the surface (34), an eccentric ramp (322), a vertex (323) and an oblique surface (324) incident with the surface (34).

4. A butterfly needle according to any one of claims 1-3, wherein the elastic means (8) is a spring.

5. A butterfly needle according to claim 4, wherein the helical spring (8) surrounds the body of the needle-holder (5); the front end of the spring (8) is integral with the needle-holder (5) because it is accommodated in a spiral seat (51) positioned adjacent to the nosepiece (54) in rear direction.

6. A butterfly needle according to any one of claims 1-5, comprising a rear cap (4) accommodated in the seat (15) at the rear end of the body (200); said rear cap (4) comprising a hole (45) which is coaxial with the hole (11) of the body (200), and comprising at the front, therefore inserted in the cavity of the hole (11), fixing means (41) for the rear end of the elastic means (8); said rear cap (4) comprising at the rear of the means (41) for fixing the elastic mean, an abutment (42) adapted to hold the rear cap (4) in the seat (15) of the body (200).

7. A butterfly needle according to any one of claims 1-6, wherein said body (200) is formed by two symmetrical half-bodies (1 and 2) which can be coupled by means of plugs (16) and corresponding pins (26); said hole (11) having, in the front part, a tapered zone (12) where the tapered front part (52) of the needle-holder (5) can be accommodated so that the needle protrudes frontally from said body (200) from the tapered zone (12) of the hole (11); said body (200) having, in the rear part, an enlarged zone with step (145) serving the function of seat (15) for the rear cap (4); wherein the step (145) serves to hold the abutment (42) of the rear cap (4).

8. A butterfly needle according to any one of claims 1-7, wherein said body (200) has, in the middle zone of the hole (11), in the upper portion of the hole (11), the guide of the nosepiece (14) adapted to guide the sliding of the nosepiece (54) of the needle-holder (5) after the mechanism is triggered; said guide (14) comprising, moving at the rear, a first part (143) parallel to the axis of the hole (11), followed by a narrowing given by a slight ramp (142), a tooth (144) and finally the seat (141), wherein the tooth (144) ensures the nosepiece (54) of the needle-holder (5) does not come out of the seat (141) once it has retracted from the elastic means (8).

9. A butterfly needle according to any one of claims 1-8, wherein said needle-holder (5) is mounted slidingly axially inside the body (200); said needle-holder (5) having a substantially cylindrical body (51) accommodated in the hole (11) of body (200); said body (51) having a tapered front part (52) from which the tip of the needle (6) protrudes; said front part (52) accommodated in the front part (12) of the body (200) when the needle is in the advanced position of use and the mechanism is blocked; said body (51) having the tapered rear part (57) which will protrude at the rear from the rear end of the body (200) and will serve to attach a flexible tube required for connecting the butterfly needle (100) to a syringe or a bottle containing the solution to be injected or intended to house the blood collected.

## Patentansprüche

1. Wegwerfbare Sicherheits-Butterflynadel mit einem Mechanismus zum Zurückziehen der Nadel (6), wobei die Nadel in einem die flexiblen Flügel (17) tragenden Hohlkörper (200) vollständig zurückgezogen ist; das Zurückziehen wird durch ein gespanntes elastisches Mittel (8) ausgeführt; wobei der Mechanismus manuell durch Bewegung einer Zunge (3) an einem Drehzapfen (7) betätigt wird, der die Zunge (3) am Körper (200) verankert; eine Bewegung, die den Bruch von mindestens einem leicht zu brechenden Mittel (135) des Körpers (200) erzeugt, wodurch der Mechanismus nach Betätigung nicht mehr aufgerüstet werden kann; wobei die Nadel (6) an einem Nadelhalter (5) montiert ist, der axial in dem Körper (200) gleitet, wobei der Nadelhalter einstückig mit dem vorderen Ende des elastischen Mittels (8) verbunden ist;
**dadurch gekennzeichnet, dass**
der Körper (200) im vorderen mittleren oberen Teil eine Mechanismuszunge (13) aufweist, die auf einer vorderen äußeren oberen Kante (19) des Körpers (200) aufliegt, wobei die Mechanismuszunge (13) zwei Löcher (131) außen und stirnseitig trägt, zur Aufnahme des Drehzapfens (7) und zum inneren Tragen eines Gehäuses (134) mit einem Blockierschutz (133) und einer Quernut an der Rückseite des Gehäuses (134), die das Anheben der Mechanismuszunge (13) ermöglicht; wobei die Mechanismuszunge mittels der Nut und mittels mindestens einer Schicht (135) einstückig mit dem Körper (200) ist, die bricht, wenn die Mechanismuszunge (13) angehoben wird;
wobei das Gehäuse (134) ein Mundstück (54) des Nadelhalters (5) mittels des Blockierschutzes (133) hält, wobei das Mundstück (54) ein Zylinder ist, der orthogonal zur Achse des Nadelhalters (5) ist, wobei das Mundstück (54) und der Blockierschutz (133) als Blockiereinrichtung des Nadelhalters in vorgeschobener Position wirken.

2. Butterflynadel nach Anspruch 1, wobei der Haupt-Hohlkörper (200) eine quadratische Außenfläche mit abgeschrägten Ecken und ein inneres axiales Loch (11) aufweist; wobei der Körper zwei Flügel (17) trägt, die nach außen vorstehen und mittels eines manuellen Griffs faltbar sind; wobei der Nadelhalter (5) in dem Loch (11) aufgenommen ist; wobei der Körper (200) im hinteren Teil Befestigungsmittel für das hintere Ende der elastischen Mittel (8) aufweist; wobei der Körper (200) Führungsmittel aufweist, die geeignet sind, die axiale Bewegung des Nadelhalters (5) innerhalb des Hohlkörpers (200) führen, und Blockier- und Streckendmittel, die geeignet sind, den Nadelhalter in einer vorgeschobenen Gebrauchsstellung zu blockieren vor der Betätigung des Mechanismus und in einer zurückgezogenen Sicherheitsposition nach der Betätigung des Mechanismus.

3. Butterflynadel nach einem der Ansprüche 1-2, bei der die Zunge (3) eine Fläche (34) umfasst, die an deren vorderem Ende und unterhalb der Fläche (34) ragend, die Drehflügel (31) aufweist, mit die jeweiligen Löcher (35), in denen der Drehzapfen (7) aufgenommen ist; wobei die Zunge (3) zwischen den Flügeln (31) und über die Oberfläche (34) ragend den Nocken (32) aufweist, der auf die Kante (19) des Körpers (200) einwirkt, indem der Nadelrückzugsmechanismus betätigt wird; wobei der Nocken (32) eine zur Oberfläche (34) orthogonale Oberfläche (321), eine exzentrische Rampe (322), einen Scheitelpunkt (323) und eine mit der Oberfläche (34) einfallende schräge Oberfläche (324) umfasst.

4. Butterflynadel nach einem der Ansprüche 1 bis 3, wobei das elastische Mittel (8) eine Feder ist.

5. Butterflynadel nach Anspruch 4, wobei die Schraubenfeder (8) den Körper des Nadelhalters (5) umgibt; das vordere Ende der Feder (8) ist einstückig mit dem Nadelhalter (5), da es in einem spiralförmigen Sitz (51) aufgenommen ist, der in der hinteren Richtung neben dem Mundstück (54) angeordnet ist.

6. Butterflynadel nach einem der Ansprüche 1 bis 5, umfassend eine hintere Kappe (4), die in dem Sitz (15) am hinteren Ende des Körpers (200) aufgenommen ist; wobei die hintere Kappe (4) ein Loch (45) umfasst, das koaxial zu dem Loch (11) des Körpers (200) ist und, an der Vorderseite daher in den Hohlraum des Lochs (11) eingesetzt, Befestigungsmittel (41) umfasst für das hintere Ende des elastischen Mittels (8); wobei die hintere Kappe (4) an der Rückseite der Mittel (41) zum Befestigen des elastischen Mittels ein Widerlager (42) aufweist, das geeignet ist, die hintere Kappe (4) in dem Sitz (15) des Körpers (200) zu halten.

7. Butterflynadel nach einem der Ansprüche 1 bis 6, wobei der Körper (200) durch zwei symmetrische Halbkörper (1 und 2) gebildet ist, die mittels Stopfen (16) und entsprechenden Stiften (26) gekoppelt werden können; wobei das Loch (11) im vorderen Teil einen verjüngten Bereich (12) aufweist, in der der verjüngte vordere Teil (52) des Nadelhalters (5) aufgenommen werden kann, so dass die Nadel stirnseitig von dem Körper (200) ausgehend von dem verjüngten Bereich (12) des Lochs (11) absteht; wobei der Körper (200) im hinteren Teil einen vergrößerten Bereich mit einer Stufe (145) aufweist, die die Funktion des Sitzes (15) für die hintere Kappe (4) erfüllt; wobei die Stufe (145) dazu dient, das Widerlager (42) der hinteren Kappe (4) zu halten.

8. Butterflynadel nach einem der Ansprüche 1 bis 7, wobei der Körper (200) im mittleren Bereich des Lochs (11) im oberen Abschnitt des Lochs (11) die Führung des Mundstücks aufweist (14), die geeignet ist, das Gleiten des Mundstücks (54) des Nadelhalters (5) zu führen, nachdem der Mechanismus ausgelöst wurde; wobei die Führung (14), indem sie zu der Rückseite bewegt, einen ersten Teil (143) parallel zur Achse des Lochs (11) umfasst, gefolgt von einer Verengung durch eine leichte Rampe (142), einen Zahn (144) und schließlich die Sitz (141), wobei der Zahn (144) sicherstellt, dass das Mundstück (54) des Nadelhalters (5) nicht aus dem Sitz (141) herauskommt, nachdem es von den elastischen Mitteln (8) zurückgezogen wurde.

9. Butterflynadel nach einem der Ansprüche 1 bis 8, wobei der Nadelhalter (5) gleitend axial innerhalb des Körpers (200) angebracht ist; wobei der Nadelhalter (5) einen im wesentlichen zylindrischen Körper (51) aufweist, der in dem Loch (11) des Körpers (200) aufgenommen ist; wobei der Körper (51) einen verjüngten vorderen Teil (52) aufweist, von dem die Spitze der Nadel (6) vorsteht; wobei der Vorderteil (52) in dem Vorderteil (12) des Körpers (200) aufgenommen ist, wenn sich die Nadel in der vorgeschobenen Gebrauchsstellung befindet und der Mechanismus blockiert ist; wobei der Körper (51) den verjüngten hinteren Teil (57) aufweist, der am Rückseite aus dem hinteren Ende des Körpers (200) herausragt und dazu dient, einen flexiblen Schlauch zu befestigen, der zum Verbinden der Butterflynadel (100) mit einer Spritze oder einer Flasche erforderlich ist, die die zu injizierende Lösung enthält oder das gesammelte Blut aufnehmen soll.

## Revendications

1. Aiguille jetable à ailettes de sécurité comprenant un mécanisme de rétractation de l'aiguille (6), dans laquelle l'aiguille est complètement rétractée à l'intérieur d'un corps creux (200) supportant les ailettes flexibles (17) ; ladite rétractation étant exécutée par un moyen élastique sous tension (8) ; ledit mécanisme étant actionné manuellement au moyen du déplacement d'une languette (3) sur un pivot (7) qui ancre la languette (3) sur le corps (200) ; un déplacement qui produit la rupture d'au moins un moyen facile à rompre (135) du corps (200), moyennant quoi le mécanisme ne peut plus être réarmé après avoir été actionné ; dans laquelle ladite aiguille (6) est assemblée sur un porte-aiguille (5) qui coulisse axialement à l'intérieur du corps (200), le porte-aiguille étant associé intégralement avec l'extrémité avant du moyen élastique (8) ;
**caractérisée en ce que**
ledit corps (200) possède, dans la partie supérieure médiane avant, une languette de mécanisme (13) reposant sur un bord supérieur externe avant (19) du corps (200), ladite languette de mécanisme (13) supportant à l'extérieur et à l'avant deux trous (131) destinés à loger le pivot (7) et supportant à l'intérieur un logement (134) comportant une protection de blocage (133) et une rainure transversale à l'arrière du logement (134) qui permet de relever la languette de mécanisme (13) ; ladite languette de mécanisme étant intégrée au corps (200) au moyen de la rainure et au moyen d'au moins une lame (135), qui se rompt lorsque la languette de mécanisme (13) est relevée ;
dans laquelle ledit logement (134) retient un embout (54) du porte-aiguille (5) au moyen de la protection de blocage (133), ledit embout (54) étant un cylindre orthogonal à l'axe du porte-aiguille (5), ledit embout (54) et ladite protection de blocage (133) jouant le rôle de moyens de blocage du porte-aiguille dans la position avancée.

2. Aiguille à ailettes selon la revendication 1, dans laquelle ledit corps creux principal (200) présente une surface extérieure carrée avec des angles biseautés et un trou axial intérieur (11) ; ledit corps supportant deux ailettes (17) faisant saillie vers l'extérieur et pouvant être pliées par une saisie manuelle ; dans laquelle ledit porte-aiguille (5) est logé dans le trou (11) ; ledit corps (200) comportant, dans la partie arrière, un moyen de fixation pour l'extrémité arrière du moyen élastique (8) ; ledit corps (200) comportant un moyen de guidage conçu pour guider le déplacement axial dudit porte-aiguille (5) à l'intérieur dudit corps creux (200), et un moyen de blocage et de fin de course conçu pour bloquer ledit porte-aiguille dans une position d'utilisation avancée avant l'actionnement du mécanisme, et dans une position de sécurité rétractée après l'actionnement du mécanisme.

3. Aiguille à ailettes selon l'une quelconque des revendications 1 et 2, dans laquelle la languette (3) comprend une surface (34) comportant, au niveau de son extrémité avant et faisant saillie sous la surface (34), les ailettes pivotantes (31) possédant les trous respectifs (35) dans lesquels le pivot (7) est logé ; ladite languette (3) comportant, entre les ailettes (31) et faisant saillie au-dessus de la surface (34), la came (32) qui agit sur le bord (19) du corps (200) en actionnant le mécanisme de rétractation de l'aiguille ; la came (32) comprenant une surface (321) orthogonale à la surface (34), une rampe excentrique (322), un sommet (323) et une surface oblique (324) incidente à la surface (34).

4. Aiguille à ailettes selon l'une quelconque des revendications 1 à 3, dans laquelle le moyen élastique (8) est un ressort.

5. Aiguille à ailettes selon la revendication 4, dans laquelle le ressort hélicoïdal (8) entoure le corps du porte-aiguille (5) ; l'extrémité avant du ressort (8) est intégrée au porte-aiguille (5) car elle est logée dans un siège en spirale (51) positionné adjacent à l'embout (54) dans la direction arrière.

6. Aiguille à ailettes selon l'une quelconque des revendications 1 à 5, comprenant un chapeau arrière (4) logé dans le siège (15) à l'extrémité arrière du corps (200) ; ledit chapeau arrière (4) comprenant un trou (45) qui est coaxial avec le trou (11) du corps (200), et comprenant à l'avant, donc inséré dans la cavité du trou (11), un moyen de fixation (41) pour l'extrémité arrière du moyen élastique (8) ; ledit chapeau arrière (4) comprenant à l'arrière du moyen (41) de fixation du moyen élastique, une butée (42) conçue pour retenir le chapeau arrière (4) dans le siège (15) du corps (200).

7. Aiguille à ailettes selon l'une quelconque des revendications 1 à 6, dans laquelle ledit corps (200) est formé par deux demi-corps symétriques (1 et 2) qui peuvent être couplés au moyen de bouchons (16) et de broches correspondantes (26) ; ledit trou (11) comportant dans la partie avant une zone conique (12) dans laquelle la partie avant conique (52) du porte-aiguille (5) peut être logée de sorte que l'aiguille fasse saillie frontalement depuis ledit corps (200) depuis la zone conique (12) du trou (11) ; ledit corps (200) comportant dans la partie arrière une zone agrandie avec une marche (145) servant de siège (15) pour le chapeau arrière (4) ; dans laquelle la marche (145) sert à retenir la butée (42) du chapeau arrière (4).

8. Aiguille à ailettes selon l'une quelconque des revendications 1 à 7, dans laquelle ledit corps (200) comporte, dans la zone médiane du trou (11), dans la partie supérieure du trou (11), le guide de l'embout (14) conçu pour guider le coulissement de l'embout (54) du porte-aiguille (5) après que le mécanisme est déclenché ; ledit guide (14) comprenant, se déplaçant à l'arrière, une première partie (143) parallèle à l'axe du trou (11), suivie d'un rétrécissement produit par une pente légère (142), une dent (144) et enfin le siège (141), dans laquelle la dent (144) garantit que l'embout (54) du porte-aiguille (5) ne sort pas du siège (141) une fois qu'il est rétracté du moyen élastique (8).

9. Aiguille à ailettes selon l'une quelconque des revendications 1 à 8, dans laquelle ledit porte-aiguille (5) est monté axialement coulissant à l'intérieur du corps (200) ; ledit porte-aiguille (5) comportant un corps sensiblement cylindrique (51) logé dans le trou (11) du corps (200) ; ledit corps (51) comportant une partie conique avant (52) depuis laquelle la pointe de l'aiguille (6) fait saillie ; ladite partie avant (52) étant logée dans la partie avant (12) du corps (200) lorsque l'aiguille est dans la position avancée d'utilisation et que le mécanisme est bloqué ; ledit corps (51) comportant la partie conique arrière (57) qui fait saillie à l'arrière depuis l'extrémité arrière du corps (200) et qui sert à fixer un tube flexible nécessaire pour relier l'aiguille à ailettes (100) à une seringue ou à une bouteille contenant la solution à injecter ou destinée à loger le sang recueilli.
